# EUROPEAN PATENT SPECIFICATION

(11) **EP 2 515 965 B1**
(45) Date of publication and mention of the grant of the patent: **15.10.2014**
(21) Application number: 10781906.2
(22) Date of filing: 25.11.2010
(51) Int. Cl.: A61M 1/30, A61M 1/36

(54) **Blood treatment device for a dialysis machine**
Blutbehandlungsvorrichtung für eine Dialysemaschine
Dispositif pour le traitement du sang pour une machine de dialyse

(30) Priority: 22.12.2009 SE 0951010; 22.12.2009 US 288881 P
(43) Date of publication of application: 31.10.2012
(73) Proprietor: Gambro Lundia AB, 220 10 Lund (SE)
(72) Inventor: NILSSON, Eddie, SE-243 32 Höör (SE); JÖNSSON, Lennart, SE-237 42 Bjärred (SE); JANSSON, Olof, SE-235 38 Vellinge (SE)
(74) Representative: Bornegard, Annette
(86) International application number: PCT/EP2010/068214
(87) International publication number: WO 2011/076511

(56) References cited:
- WO-A1-2005/061043
- WO-A1-2009/127626
- DE-A1- 3 328 744
- US-B2- 6 645 166

## Description

### Technical Field

The invention relates to a blood treatment device and a dialysis machine for extracorporeal blood treatment. The blood treatment device may be used for double-needle and/or single-needle dialysis.

### Background Art

Today various dialysis methods are known, which include so called double-needle dialysis (DN) and single-needle dialysis (SN). In double-needle dialysis blood is withdrawn from a patient via one needle and returned to the patient via another needle. In this case, the blood is pumped continuously through a dialyzer which renders a steady flow of blood from and to e.g. a patient. In single-needle dialysis only one needle is required in order to withdraw and return blood, which often is advantageous in a self-care setting since there is no risk for dislodgement of a blood return needle and thereby loss of blood being pumped out unintentionally via a blood withdrawal needle. Also, when using single-needle dialysis, fewer needle punctures for accessing the patient's blood are required relative to double-needle treatment. However since single-needle dialysis withdraws and returns blood cyclically via the same needle, more time is required for completing the dialysis in comparison with the double-needle dialysis.

Accordingly, double-needle dialysis has both advantages and drawbacks in comparison with single-needle dialysis, and vice versa. For this reason it is desirable to offer blood treatment devices and dialysis machines that allow both double-needle and single-needle operation.

US6645166 discloses such a blood treatment device, which permits both single-needle and double-needle operation. The device has a blood treatment unit, in particular a dialyzer that has an inlet connected to a feed line and an outlet connected to a return line. The feed line has two parallel line branches with a positive displacement pump being connected into the first and second line branches. To produce a fluid connection between the outlet of the dialyzer and one of the two pumps, a connection line is provided. For double-needle operation, the feed and return lines are connected to an arterial and a venous needle. For single-needle operation, the feed and return lines are brought together and connected to a common needle.

The blood treatment device described above is capable of offering both double- and single-needle operation. However, the device appears relatively complex in terms of efficiently controlling a flow of blood trough the apparatus, as well as appears complex in terms of allowing an efficient manufacturing process of the device.

### Summary

It is an object of the invention to at least partly overcome one or more of the above-identified limitations of the prior art. In particular, it is an object to provide a blood treatment device that offers more efficient control of a flow of blood in the device, which control may be used for double-needle, single-needle and/or combined double- and single-needle dialysis.

Hence, according to a first aspect a blood treatment device as defined in claim 1 is provided, which comprises: a blood treatment unit having an inlet and an outlet; a blood withdrawal line connected to the inlet of the blood treatment unit, the blood withdrawal line having a first branch line and a second branch line connected in parallel with the first branch line; a blood return line connected to the outlet of the blood treatment unit; a first pump connected in the first branch line such that the first branch line is divided into an upstream section and a downstream section; and a second pump connected in the second branch line such that the second branch line is divided into an upstream section and a downstream section. The blood treatment device also comprises a valve configured to: open the upstream section and close the downstream section of the first branch line, and close the upstream section and open the downstream section of the second branch line, when the valve is moved to a first position; and close the upstream section and open the downstream section of the first branch line, and open the upstream section and close the downstream section of the second branch line, when the valve is moved to a second position.

For the blood treatment device, the various "lines" (e.g. blood with-drawal lines, branch line etc.) does not necessarily have to be blood lines that have an elongated extension, but may also represent a respective connection along a blood line where the connection forms a boundary between two or more entities in the blood line. Even though such a connection does not have a physical extension, it enables a flow of blood between entities and may hence bee seen as a "line" (e.g. a blood withdrawal line, branch line etc., depending on which functionality the connection realizes). However, several or all of the lines in the blood treatment device may be elongated, such as in the form of a closed channel or tube, which allows for more versatile mutual arrangement of the various parts of the blood treatment device.

The blood treatment device is advantageous in that blood flowing in the device may be efficiently controlled by virtue of the valve, which valve may be a unitary valve. The efficient control is associated with both the valve and the pumps of the blood treatment device and may provide e.g. a more smooth transition between the pump phases (phases of drawing in blood and feeding blood). Also, the control of the flow of blood in the blood treatment device requires relatively few control signals or control operations to be provided to the blood treatment device, for example from a dialysis machine arranged to use the blood treatment device in a dialysis operation. Thus, the blood treatment device may enable simpler implementation of a dialysis machine.

Moreover, it has surprisingly been found that the blood treatment device facilitates more efficient manufacturing, since relatively few and/or more easily exchangeable components may be used for assembling the blood treatment device in accordance with single-needle, double-needle or combined double/single-needle operation.

The valve may be configured to provide double-needle dialysis, single-needle dialysis or combined double/single-needle dialysis. In the latter case either double-needle operation or single-needle operation of the blood treatment device may be selected by an operator prior a dialysis operation is commenced. In the former cases, i.e. for double-needle dialysis and single-needle dialysis, the mode of operation is typically determined during assembly of the blood treatment device.

The valve may comprise a valve body and a valve seat in which the valve body is arranged. The valve body may typically be a unitary valve body.

The valve may comprise a closure element connected to the valve seat, such that the valve body is enclosed by the valve seat and the closure element, which may facilitates convenient replacement of the valve body as well as versatile assembly of the blood treatment device, by using different valve bodies enclosed by the valve seat and closure element.

The valve may be configured to move between the first position and the second position by rotation of the valve. The valve may have a circular shape. Alternatively or additionally, the valve may move between the two positions by linear movement. Also, linear and rotational movement may be combined, in which case the valve may, for example, be moved between the first and the second position and between a third and a fourth position by rotation, while the valve may be linearly moved between the first or second position to the third or fourth position, and vice versa.

The valve may comprise an engagement member configured to interact with an actuator of a dialysis apparatus, for allowing the valve to be moved between the first position and the second position. The shape and type of the engagement member typically depend on the type of actuator that is used. For example, the actuator may be a an electromechanical device such as a step-motor, in which case the engagement member may have the form a mechanical connector capable of cooperating with the actuator, such that a physical movement of the actuator is transferred to the engagement member. The actuator may also be a pressure controlled switch that applies a pressure on the engagement member (or on a part of the actuator connected to the engagement member), for moving the valve between its positions.

The valve may comprise: a first conduit configured to provide the opening and closing of the upstream sections of the first and second branch lines; and a second conduit configured to provide the opening and closing of the downstream sections of the first and second branch lines. The conduits may, for example, have the form of a respective recess, channel, slot, groove or similar cut-out or in or penetration through the valve.

The valve may comprise a third conduit arranged in the blood return line. This may be useful if, for example, the valve shall operate in further positions that defines other modes of dialysis.

The valve body may comprise the first and second conduits.

The valve may comprise at least six openings, wherein each of a blood inlet of the blood treatment device, pump inlets and pump outlets of the pumps, and the inlet of the blood treatment unit are connected to a respective opening of the openings. Typically the openings are arranged to allow a flow of blood through one or more of the openings, in dependence of a desired mode of operation of the blood treatment device and typically also in dependence of phases of the mode of operation. The openings may be seen as inlets or connection points that may allow a flow of blood in the blood treatment device. The six openings may typically facilitate the control of blood in accordance with double-needle operation of the blood treatment device.

The valve may comprise two further openings, wherein each of the outlet of the blood treatment unit and a blood outlet of the blood treatment device are connected to a respective opening of the further openings. The two further openings are similar with the six openings described above, but may be used for allowing the blood treatment device to operate in a single-needle mode.

The valve seat may comprise the six openings. The valve seat may also comprise the two further openings.

The valve may be configured to: disconnect the second branch line from the first branch line and connect the second pump in the blood return line, such that the blood return line is divided into an upstream section and a downstream section, when the valve is in any of a third position and a fourth position; open the upstream section of the first branch line and close the downstream section of the blood return line, when the valve is moved to the third position; and close the upstream section of the first branch line and open the downstream section of the blood return line, when the valve is moved to the fourth position.

The disconnection of the branch line and the connection of the second pump is typically performed by the valve (by changing flow paths of the blood), and result in a single-needle configuration of the blood treatment device.

The valve may comprise a conduit configured to provide opening and closing of the downstream section of the blood return line. This conduit and its implementation may be similar with the conduits described above.

The blood treatment unit may have an elongated shape, and the valve may be arranged at a long side of the blood treatment unit. This arrangement has shown practical in terms of providing an efficient control of the blood treatment device, particularly in terms of accessing a control point of the valve.

Each of the first pump and the second pump may be arranged at a long side of the blood treatment unit. This arrangement is advantageous e.g. in that the valve more easily accesses and thus controls the flow of blood to or from the pumps.

Each of the first pump and the second pump may be membrane pumps.

According to a second aspect of the invention, a blood treatment device as defined in claim 18 is provided, which comprises: a blood treatment unit having an inlet and an outlet; a blood withdrawal line connected to the inlet of the blood treatment unit; a blood return line connected to the outlet of the blood treatment unit; a first pump connected in the blood withdrawal line such that the blood withdrawal line is divided into an upstream section and a downstream section; and a second pump connected in the blood return line such that the blood return line is divided into an upstream section and a downstream section. The blood treatment device according to this second aspect also comprises a valve configured to: open the upstream section of the blood withdrawal line and close the downstream section of the blood return line, when the valve is moved to a third position; and close the upstream section of the blood withdrawal line and open the downstream section of the blood return line, when the valve is moved to a fourth position.

The blood treatment device according to the second aspect renders a different flow of blood, i.e. a flow that is typical for single-needle operation, but is still based on the same principles as the blood treatment device according to the first aspect. Accordingly, it shares the corresponding advantages and may implement any of the previously described features, but with the difference that it may be adapted to perform double-needle operation in addition to the single needle operation, even though this is not necessary.

According to a third aspect of the invention, a blood treatment device as defined in claim 35 is provided, which comprises: a blood treatment unit having an inlet and an outlet; a blood withdrawal line configured to receive blood; a blood return line configured to return blood; a first pump having an inlet and an outlet; and a second pump having an inlet and an outlet. The blood treatment device according to this aspect also has a valve seat which comprises at least six openings, wherein each of the inlet of the blood treatment unit, the blood withdrawal line, the inlet and outlet of the first pump, and the inlet and the outlet of the second pump are connected to a respective opening of the six openings, for allowing a valve body arranged in the valve seat to control a flow of blood via at least two of the six openings.

The valve seat in the blood treatment device according to the third aspect may comprise two further openings, wherein each of the blood return line and the blood treatment unit are connected to a respective opening of the further openings.

The blood treatment devices according to the third aspect may, when six openings are provided, be arranged to perform double-needle operation, for example by using a suitable valve body. In case eight openings are provided, the blood treatment device may also operate in single-needle mode if another valve body is used. Optionally, if still another type of valve body is used, the blood treatment device may operate in either double- or single-needle mode. The blood treatment devices according to the third aspect may incorporate any of the features described in connection with the previously disclosed blood treatment devices, and share the corresponding advantages.

According to another aspect of the invention, a blood treatment apparatus is provided, which comprises any of the blood treatment devices described above.

Still other objectives, features, aspects and advantages of the invention will appear from the following detailed description, from the attached claims as well as from the drawings.

### Brief Description of the Drawings

Embodiments of the invention will now be described, by way of example, with reference to the accompanying schematic drawings, in which
Fig. 1 illustrates a blood treatment apparatus which includes a blood treatment device according to an embodiment of the invention,
Fig. 2a is an exploded view the blood treatment device illustrated in Fig. 1,
Fig. 2b is an exploded view corresponding to Fig. 2a, but seen from another side of the blood treatment device,
Fig. 3a is a cross-sectional view of the blood treatment device of Fig. 1,
Fig. 3b is an enlarged, cross-sectional view of a valve seat of the blood treatment device of Fig. 1,
Fig. 4a is an enlarged, partial view of section A in Fig. 3a, where the blood treatment device operates in a first phase during double-needle dialysis,
Fig. 4b is a principal view of the blood treatment device in operation according to Fig. 4a,
Fig. 5a is an enlarged, partial view of section A in Fig. 3a, where the blood treatment device operates in a second phase during double-needle dialysis,
Fig. 5b is a principal view of the blood treatment device in operation according to Fig. 5a,
Fig. 6a is an enlarged, partial view of section A in Fig. 3a, where the blood treatment device operates in a first phase during single-needle dialysis,
Fig. 6b is a principal view of the blood treatment device in operation according to Fig. 6a,
Fig. 7a is an enlarged, partial view of section A in Fig. 3a, where the blood treatment device operates in a second phase during single-needle dialysis, and
Fig. 7b is a principal view of the blood treatment device in operation according to Fig. 7a.

### Detailed description

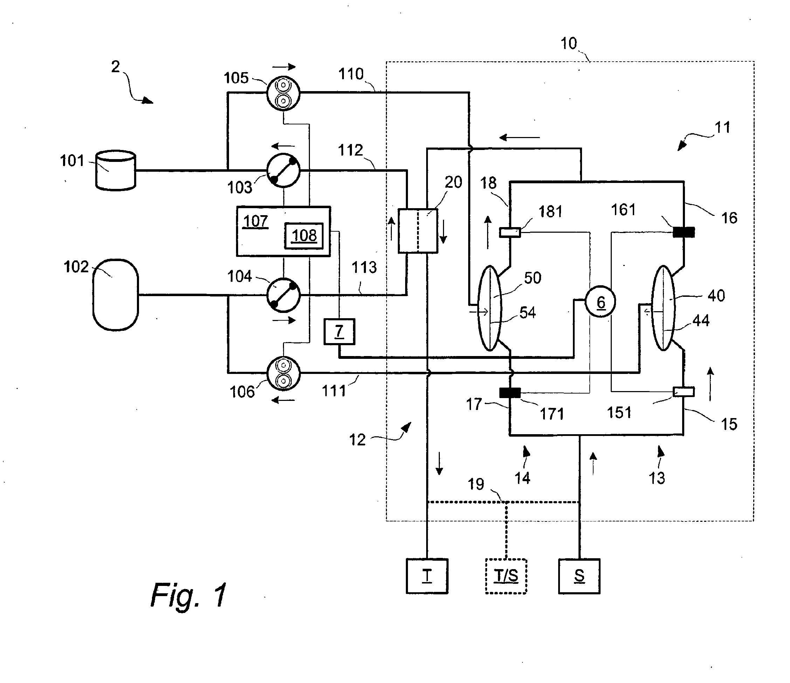

With reference to Fig. 1, a blood treatment apparatus 2 (apparatus) is illustrated which includes a blood treatment device 10. The apparatus 2 is typically a dialysis machine capable of performing blood treatment or treatment of other similar body fluids, and has a fluid withdrawal line 113 in which a first fluid pump 104 is arranged and a fluid return line 112 in which a second fluid pump 103 is arranged. The fluid withdrawal line 113 is connected to a fluid inlet of a blood treatment unit 20 (typically a dialyzer) of the blood treatment device 10, while the fluid return line 112 is connected to an outlet of the blood treatment unit 20.

The first fluid pump 104 is connected to a reservoir compartment 102 and the second fluid pump 103 is connected to a sink 101, and the pumps 104, 103 are arranged to generate a flow of treatment fluid (dialysis fluid) from the reservoir compartment 102, through the blood treatment unit 20 and to the sink 101 that receives used treatment fluid. In this context, the flow of treatment fluid has a flow direction from the reservoir compartment 102 towards the sink 101, where "upstream" refers to a position further up the flow, and "downstream" refers to a position further down the flow, as seen from a certain point along the flow of treatment fluid. For example, the first pump 104 is arranged upstream the blood treatment unit 20 while the second pump 103 is arranged downstream the blood treatment unit 20. The corresponding relation between "upstream" and "downstream" applies for all components arranged along any type of line or conduit in which a flow is present.

An end of a first control line 111 is connected to the fluid withdrawal line 113 at a position downstream the reservoir compartment 102 and upstream the first fluid pump 104, while an opposite end of the first control line 111 is connected to a fluid inlet of a first blood pump 40. A first control pump 106, such as a gear pump or a peristaltic pump, is connected in the first control line 111. In a similar manner an end of a second control line 110 is connected to the fluid return line 112 at a position downstream the second fluid pump 103 and upstream the sink 101, while an opposite end of the second control line 110 is connected to a fluid inlet of a second blood pump 50. A second control pump 105 is connected in the second control line 110, and may be of the same type as the first control pump 106. The control lines 111, 110 are connected to the fluid lines 113, 112 for purpose of receiving a treatment fluid that may be used for controlling the blood pumps 40, 50. Instead of being connected to the fluid lines 113, 112, the control lines 111, 110 may be connected to e.g. the same fluid line, such as to the fluid return line 112 downstream the second fluid pump 103. Alternatively, the control lines 111, 110 can be connected to another part of the fluid lines 113, 112, or to a separate fluid line. The separate fluid line may be connected to a source of working fluid that is different from the treatment fluid.

The first blood pump 40 and second blood pump 50 are parts of the blood treatment device 10, just like the blood treatment unit 20, and the blood treatment device 10 is generally easily exchanged for a new (similar or different) blood treatment device.

The blood treatment device 10 has a blood withdrawal line 11 and a blood return line 12 for drawing blood from a blood source S, passing the drawn blood through the blood treatment unit 20, and returning the blood to a target vessel T. For this purpose the blood withdrawal line 11 is connected to a blood inlet of the blood treatment unit 20 while the blood return line 12 is connected to a blood outlet of the blood treatment unit 20.

The apparatus 2 may be operated in a double-needle mode or in a single-needle mode, where the double-needle mode is divided into two phases that are referred to as a first phase DN-P1 and a second phase DN-P2, and where the single-needle mode is divided into two phases that are referred to as a third phase SN-P1 and a fourth phase SN-P2. The four phases are mutually different and are described in detail below.

For purpose of illustrating the blood treatment device 10 in connection with the apparatus 2, operation according to the first phase DN-P1 is shown in Fig. 1, where the blood withdrawal line 11 is divided into a first branch line 13 and a second branch line 14 that is arranged in parallel with the first branch line 13.

The first blood pump 40 is arranged in the first branch line 13, where the first blood pump 40 divides the first branch line 13 into an upstream section 15 and a downstream section 16. The second blood pump 50 is arranged in the second branch line 14, where the second blood pump 50 divides the second branch line 14 into an upstream section 17 and a downstream section 18. When arranged in the respective branch line, a blood inlet of the first blood pump 40 is connected to section 15 while a blood outlet of the first blood pump 40 is connected to section 16. In a similar manner a blood inlet of the second blood pump 50 is connected to section 17 while a blood outlet of the second blood pump 50 is connected to section 18.

The first blood pump 40 may be a membrane pump with a fluid chamber and a blood chamber. More particularly, the fluid chamber of the first blood pump 40 is connected to the first control line 111, such that treatment fluid may enter and exit the fluid chamber of the first blood pump 40. The blood chamber of the first blood pump 40 is connected to the blood inlet and outlets of the pump 40. The fluid chamber constitutes the working chamber of the pump 40, such that blood enters the blood chamber when the fluid exits the fluid chamber, and vice versa. The corresponding situation applies for the second blood pump 50, which has its fluid chamber connected to the second control line 110.

Since Fig. 1 illustrates double-needle operation of the apparatus 2, blood is withdrawn from the blood source S and is returned to the target vessel T via a respective connection. The blood source S and the target vessel T may be containers of blood but may also represent the vascular system of a patient. In the latter case, the blood source S may be a blood access of a patient, such as an fistula, graft or artery of the patient while the target vessel T may be e.g. a fistula, graft or vein of the patient.

However, in case the apparatus 2 operates in a single-needle mode, the blood is withdrawn and returned at a combined blood source and target vessel S/T (illustrated in dashed lines), instead of withdrawn respectively returned to a separate blood source/target vessel. In the single-needle operation the blood withdrawal line 11 and the blood return line 12 are connected to the combined blood source and target vessel S/T via e.g. a Y-connection piece 19 (illustrated in dashed lines).

The blood treatment device 10 also includes a valve 6 arranged to control a flow of blood through each of the upstream 15 and downstream 16 sections of the first branch line 13 and each of the upstream 17 and downstream 18 sections of the second branch line 14. The valve 6 may, as will be described in detail below, be a unitary valve that controls the flow of blood in each of the sections 15, 16, 17, 18. This control is functionally illustrated by control points 151, 161, 171, 181, where an outlined control point indicates that a flow of blood is possible through the corresponding section, such as control point 151 in section 15 and control point 181 in section 18, while a "dark" control point indicates that a flow of blood through the corresponding section is blocked, such as control point 161 in section 16 and control point 171 in section 17. For allowing the valve 6 to perform the multiple control (i.e. controlling several control points), the apparatus 2 includes an actuator 7 that is connected to the valve 6, and that may switch the valve 6 for operation in accordance with any of the four phases DN-P1, DN-P2, SN-P1, SN-P2. The actuator 7 may, for example, be an electrical step-motor or any other controllable device suitable for moving a relatively small device between two or more positions in a cyclic manner.

In Fig. 1 as well as in subsequent figures, various flow directions for treatment fluid and blood are indicated by arrows at the relative item where the flow occurs, with the head of the arrow indicating the flow direction. For example, the arrow at the control point 151 indicates that a flow of blood is present via the control point 151, in a direction from the blood source S to the first blood pump 40.

The first fluid pump 104, the second fluid pump 103, the first control pump 106, the second control pump 105 and the actuator 7 are each connected to and controlled by a control unit 107 that is arranged in the apparatus 2. The pumps 103-106 may be e.g. peristaltic pumps or gear pumps or any other conventional pump capable of generating (and possible also blocking) a flow of treatment fluid. For performing the control of the pumps 104-106 and the actuator 7 (and hence the valve 6 via the actuator 7) the control unit 107 has a processing unit 108, such as a conventional central processing unit, a microprocessor or any similar control circuit. For performing the control of the pumps and actuator/valve as well as other standard control operations of the apparatus 2, the control unit 107 comprises a computer-readable medium (not shown) that stores processing instructions which, when executed by the processing unit 107, performs the operations described below, or more particularly, performs operation of the apparatus 2 in accordance with any of the four phases DN-P1, DN-P2, SN-P1, SN-P2.

The processing instructions, i.e. a computer program code for carrying out the operation of the apparatus 2 may for development convenience be written in a high-level programming language such as Java, C, and/or C++ but also in other programming languages, such as, but not limited to, interpreted languages. The software instructions may also be written in assembly language or even micro-code to enhance performance and/or memory usage. It will be further appreciated that the functionality of any or all of the functional steps performed by the apparatus may also be implemented using discrete hardware components, one or more application specific integrated circuits, or a programmed digital signal processor or microcontroller.

The computer readable medium that stores the processing instructions may be any suitable computer-readable medium, such as a RAM, ROM or Flash memory, a solid-state-drive a magnetic disk (hard-drive), a CD or a DVD.

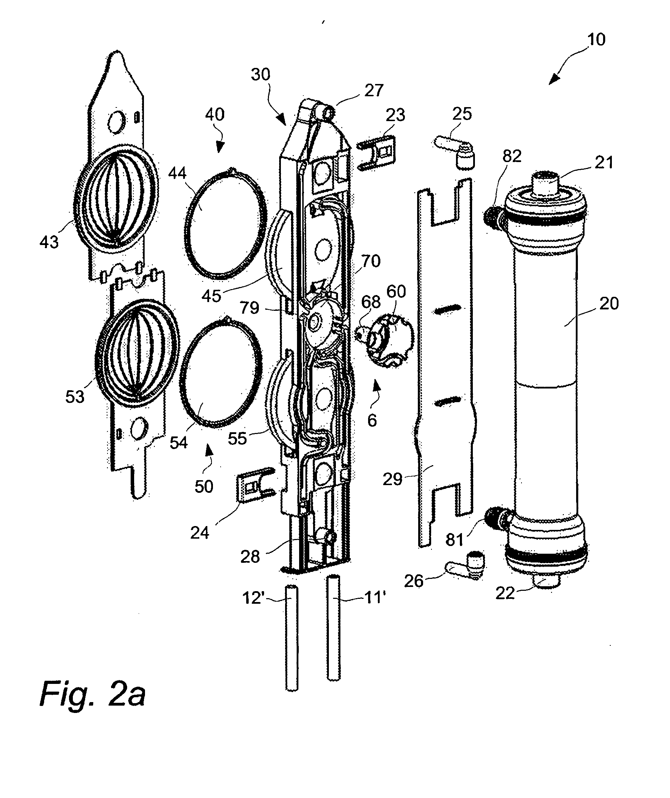

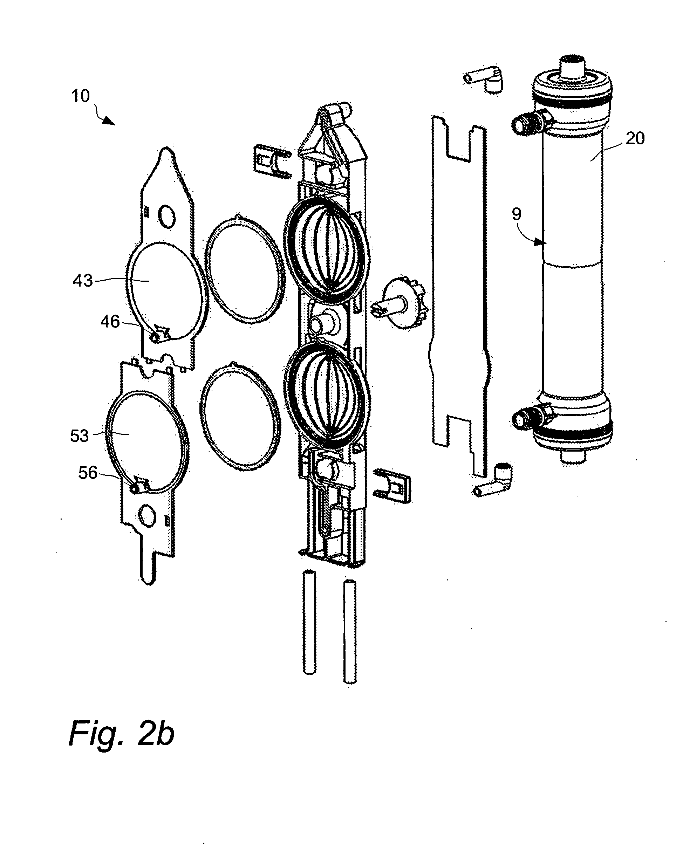

With reference to Figs 2a and 2b the blood treatment device 10 is illustrated in further detail. The blood treatment device 10 is typically a disposable device in form of a single unit that is used once for blood treatment of a certain patient or container of blood, and is disposed of when the treatment is completed. When treatment of the same or another patient or container of blood shall commence, a new blood treatment device 10 is connected to the fluid lines 112, 113, to the control lines 110, 111 and to the actuator 7. Of course, instead of connecting a new blood treatment device to the apparatus, the used blood treatment device may be cleaned for allowing reuse, for example by using existing sterilization and cleaning techniques.

As mentioned, the blood treatment device 10 has a first blood pump 40 and a second blood pump 50. The first blood pump 40 has a fluid chamber part 43 that is connected to a blood chamber part 45. Between the fluid chamber part 43 and the blood chamber part 45 a membrane 44 is arranged, such that a blood chamber is defined by the membrane 44 and the blood chamber part 43. In a corresponding manner the membrane 44 and the fluid chamber part 43 define a fluid chamber which constitutes the so called working chamber for pumping blood through the blood chamber. In a similar manner the second blood pump 50 of the blood treatment device 10 also has a fluid chamber part 53, a blood chamber part 55 and a membrane 54 arranged in between, such that the second blood pump 50 also has a fluid chamber and a blood chamber.

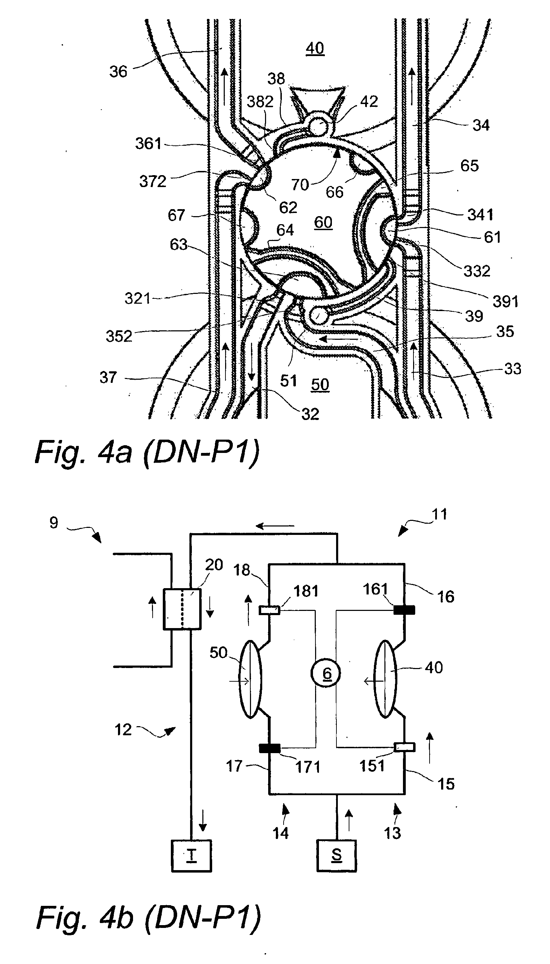

The membranes 44, 54 are illustrated in Fig.1 as well as in subsequently described Figs 4b, 5b, 6b and 7b, where the movement of the respective membrane 44, 54 is illustrated with an arrow.

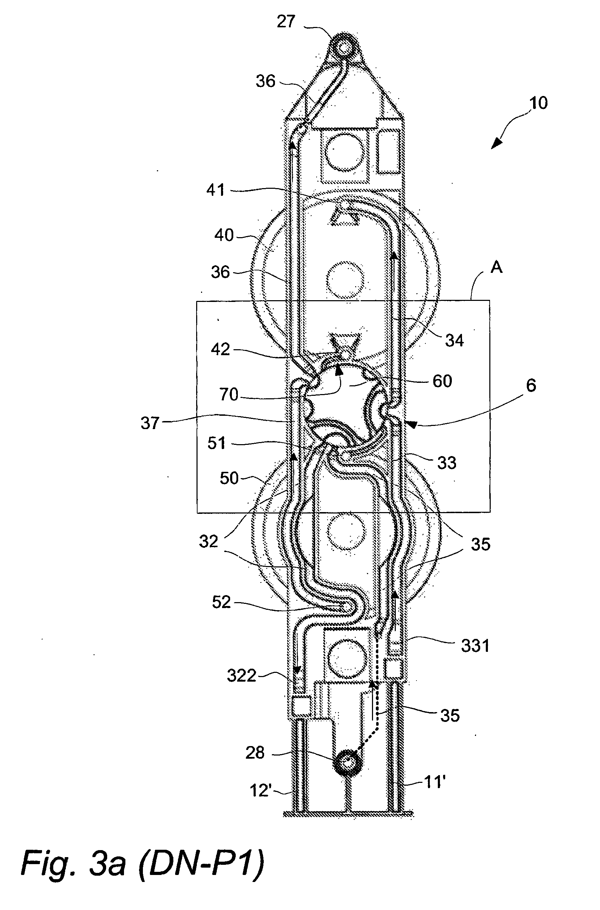

As, can be seen in Fig. 2b, each of the fluid chamber parts 43, 53 has a respective inlet 46, 56 connected to the associated control line 111 or 112 for allowing fluid to enter and exit the fluid chamber, such that the respectively associated membrane may be moved in a cyclic manner for increasing the volume of the fluid chamber (thereby pushing out blood from the blood chamber) and decreasing the volume of the fluid chamber (thereby drawing in blood into the blood chamber). As mentioned and with further reference to Fig. 3a, the first blood pump 40 has a pump inlet 41 and a pump outlet 42 while the second blood pump 50 has a pump inlet 51 and pump outlet 52 for drawing in and pushing out blood from the blood pumps 40, 50.

Returning to Figs 2a and 2b, the blood chamber parts 45, 55 are included in a base section 30 of the blood treatment device 10. The base section 30 has a valve seat 70 which together with a valve body 60 and a closure element 29 form the valve 6. For fitting the valve body 60 into the valve seat 70, the body 60 has an engagement member 68 in form of a protrusion that is inserted into an opening 79 in the seat 70. The engagement member 68 has, for example and as may bee seen, a slot that may engage with the actuator 7. The closure element 29 is attached to the base section 30, such that the valve body 60 is enclosed by the closure element 29 and the valve seat 70 of the base section 30.

The blood treatment unit 20 has a blood inlet 21, a blood outlet 22, a fluid inlet 81 and a fluid outlet 82, and has an elongated, circular shape with a long side 9 facing the closure element 29. The fluid inlet 81 and fluid outlet 82 also face and protrude trough the closure element 29, the base section 30 and the fluid chamber parts 43, 53. The various parts are brought together during assembly and are held together by two clips 23, 24 that are snapped on the fluid inlet and outlet 81, 82. The assembled blood treatment device 20 may thus be a unitary blood treatment device. As shown, each of the chamber parts 43, 53 has tabs opposite a respective opening for the inlet/outlet 81, 82, which tabs engage in the base section 30 such that the chamber parts 43, 53 are firmly fixed. Of course, the chamber parts 43, 53 may also be made as one unit. Preferably, when the blood treatment unit 20 is assembled, the engagement member 68 may extend towards and through the chamber parts 43, 53 along the same direction as the fluid inlets 46, 56.

The base section 30 has a number of blood lines, which will be described in detail, that direct the blood via the valve 6. For allowing the blood to enter the blood lines the base section 30 comprises an inlet section 11' of the blood withdrawal line 11 and an outlet section 12' of the blood return line 12. In case the blood treatment device 10 is configured for double-needle operation, the inlet section 11' is connected, generally via a flexible tube, to a needle configured to be inserted in e.g. a patients blood access or to a connection coupled to a blood source, while the outlet section 12' is connected, generally via a flexible tube, to a needle configured to be inserted in e.g. a vein of the patient or to a connection coupled to a target vessel arranged to receive treated blood. In case the blood treatment device 10 is configured for singe-needle operation, as will be described below, the sections 11' and 12 " are connected to a common needle, for example via the Y-connection piece 19 (shown in Fig. 1).

Blood that enters the blood treatment device 10, or more specifically enters the base section 30, exits the base section 30 from a blood outlet 27 and enters via a first connection piece 25 the inlet 21 of the blood treatment unit 20. After blood has entered the inlet 21 and passed through the blood treatment unit 20 the blood exits via the outlet 22 and re-enters the base section 30 via a second connection piece 26 through an inlet 28 of the base section 30.

With reference to Fig. 3a the base section 30 has a first blood line 33, a second blood line 34, a third blood line 37, a fourth blood line 36, a fifth blood line 35, a sixth blood line 32, a seventh blood line 38 and an eighth blood line 39.

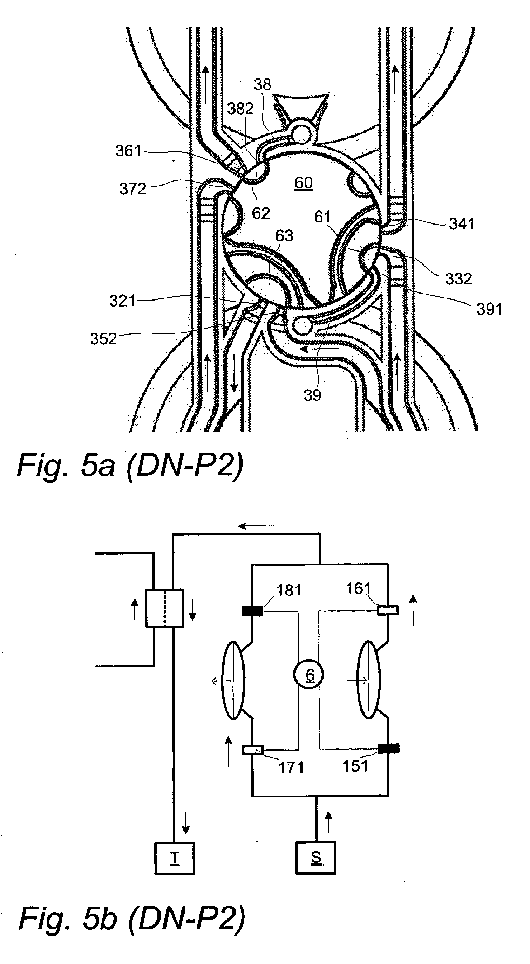

With further reference to Fig. 4a, which is an enlarged view of section A in Fig. 3a (just like Figs 5a, 6a and 7a subsequently described but for other phases), the first blood line 33 has a first end 331 (shown in Fig 3a) connected to the section 11' and a second end 332 connected to the valve seat 70. The second blood line 34 has a first end 341 connected to the valve seat 70 and a second end connected to the inlet 41 of the first blood pump 40. The third blood line 37 has a first end connected to the outlet 52 of the second blood pump 50 and a second end 372 connected to the valve seat 70. The fourth blood line 36 has first end 361 connected to the valve seat 70 and a second end connected to the blood outlet of the base section 30, which means that the second end of the fourth blood line 36 is connected to the inlet 21 of the blood treatment unit 20. The fifth blood line 35 has a first end connected to the blood inlet 28 of the base section 30, which means that the first end of the fifth blood line 35 is connected to the outlet 22 of the blood treatment unit 20, and has a second end 352 connected to the valve seat 70. The sixth blood line 32 has a first end 321 connected to the valve seat 70 and a second end 322 connected to the section 12'. The seventh blood line 38 has a first end connected to the outlet 42 of the first blood pump 40 and a second end 382 connected to the valve seat 70. Finally, the eighth blood line 39 has a first end 391 connected to the valve seat 70 and a second end connected to the inlet 51 of the second blood pump 50.

The valve body 60 ha a first conduit 61, a second conduit 62, a third conduit 63, a fourth conduit 64, a fifth conduit 65, a sixth conduit 66 and a seventh conduit 67. The first, second, third, sixth and seventh conduits 61, 62, 63, 66, 67 may be formed by a respective recess in a periphery of the valve body 60 that faces the valve seat 70. The fourth and fifth 64, 65 conduits may be formed as a respective channel in the valve body 60 with an inlet and an outlet at the periphery of the valve body 60. Of course, it is possible to re-arrange the conduits in the valve body 60 such that one or more of the conduits form a recess instead of a channel, and vice versa.

The valve body 60 and the valve seat 70 are in this embodiment circular such that the valve body 60 may rotate between four positions, as controlled by the control unit 107 via the actuator 7, which positions correspond to the four phases DN-P1 (the first position), DN-P2 (the second position), SN-P1 (the third position) and SN-P2 (the fourth position).

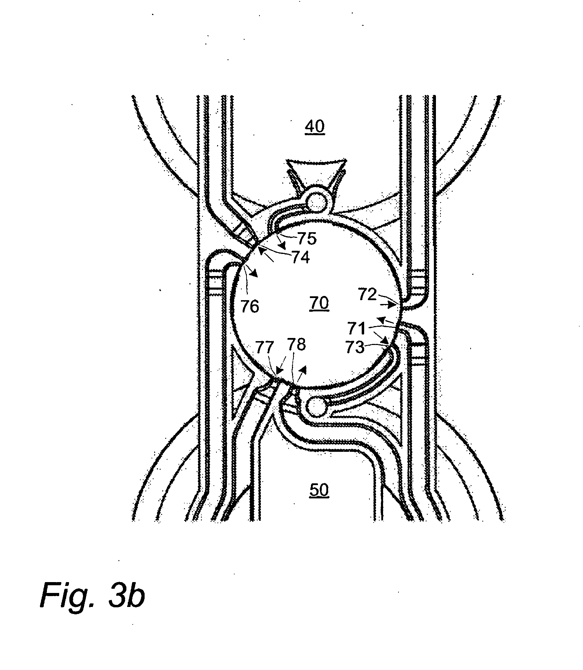

The valve body 60 is arranged in the valve seat 70 with a clearance that is sufficient for allowing the rotational movement while still preventing a leakage of blood from one conduit in the valve body 60 to another. The ends of the eight blood lines 32-39 that are connected to the valve seat 70 enters the valve seat 70 via a respective opening 71-78, as shown in Fig. 3b. As described above, flow directions for blood in the blood treatment device 10 are indicated by arrows.

When viewing the blood treatment device 10 illustrated in Figs 2a and 2b in connection with the apparatus 2 of Fig. 1, the inlet 46 of the first blood pump 40 is connected to the first control line 111, the inlet 56 of the second blood pump 50 is connected to the second control line 110 while the actuator 7 is connected to the valve body 60 via the slot in the engagement member 68. Also, the fluid inlet 81 of the blood treatment unit 20 is connected to the fluid withdrawal line 113 and the fluid outlet 82 of the blood treatment unit 20 is connected to the fluid return line 112.

The fluid chambers may however have a respective fluid inlet and fluid outlet instead of a combined inlet/outlet, and the apparatus 2 may have a set of control valves for increasing and decreasing the volume of the fluid chamber.

With reference to Figs 4a and 4b, operation of the apparatus 2 in the first phase of double-needle treatment (DN-P1) is illustrated. It should here be noted that Fig. 4b (as well as Figs 5b, 6b and 7b subsequently described) is a principal view of the operation of the valve 6, which means that e.g. the actuator 7 and control lines 111, 110 are connected to the valve 6 and blood pumps 40, 50, even though this is not illustrated. Also, in double-needle operation the inlet section 11' of the blood withdrawal line 11 is connected to the blood source S and the outlet section 12' of the blood return line 12 is connected to the target vessel T.

In the first phase of double-needle treatment DN-P1 the control unit 107 sets the valve body 60 to the first position, such that the first conduit 61 faces the second end 332 of the first blood line 33 and faces the first end 341 of the second blood line 34, for allowing a flow of blood from the first blood line 33 to the second blood line 34 via the first conduit 61. This means that the blood source S is in fluid communication with inlet 41 of the first blood pump 40, which corresponds to the control point 151 being open.

Simultaneously, the second conduit 62 faces the first end 361 of the fourth blood line 36 and faces the second end 372 of the third blood line 37, for allowing a flow of blood from the third blood line 37 to the fourth blood line 36 via the second conduit 62. This means that the outlet 52 of the second blood pump 50 is in fluid communication with the inlet 21 of the blood treatment unit 20, which corresponds to the control point 181 being open.

Moreover, the third conduit 63 faces the second end 352 of the fifth blood line 35 and faces the first end 321 of the sixth blood line 32, for allowing a flow of blood from the fifth blood line 35 to the sixth blood line 32. This means that the outlet 22 of the blood treatment unit 20 is in fluid communication with the outlet section 12' of the blood return line 12.

As shown in Fig. 4a, in the first phase of double-needle treatment DN-P1 the second end 382 of the seventh blood line 38 and the first end 391 of the eighth blood line 39 are blocked by the peripheral boundary of the valve body 60. This means that no blood may exit from the outlet 42 of the first blood pump 40 or may enter the inlet 51 of the second blood pump 50, which corresponds to the control points 161 and 171 being closed.

During each phase DN-P1, DN-P2 of double-needle treatment the first fluid pump 104 and the second fluid pump 103 continuously generate a flow of treatment fluid from the reservoir compartment 102 to the sink 101, which allows the control pumps 105, 106 to feed or withdraw treatment fluid from the blood pumps 40, 50. More precisely, in the first phase of double-needle treatment DN-P1 the first control pump 106 draws treatment fluid from the fluid chamber of the first blood pump 40, such that blood fills the blood chamber of the first blood pump 40. At the same time the second control pump 105 feeds treatment fluid to the fluid chamber of the second blood pump 50, such that blood leaves the blood chamber of the second blood pump 50. Accordingly, by virtue of the control pumps and the first position of the valve, a flow of blood in the blood treatment unit 20 is generated as illustrated by Figs 4a and 4b, i.e. the first blood pump 40 draws blood from the blood source S and the second blood pump 50 feeds blood to the blood treatment unit 20 and further to the target vessel T.

When relating the blood lines of Fig. 4a to the flow lines of Fig. 4b, it may in functional terms be said that the second blood line 34 in combination with the first blood line 33 correspond to upstream section 15, while the seventh blood line 38 in combination with the fourth blood line 36 correspond to downstream section 16. In a similar manner the eighth blood line 39 in combination with the first blood line 33 functionally correspond to section 17, while the third blood line 37 in combination with the fourth blood line 36 functionally correspond to downstream section 18. The fifth blood line 35 and the sixth blood line 32 functionally correspond to the blood return line 12.

With reference to Figs 5a and 5b, operation of the apparatus 2 in the second phase of double-needle treatment (DN-P2) is illustrated. In this case the control unit 107 sets the valve body 60 to the second position, such that the first conduit 61 faces the first end 391 of the eighth blood line 39 instead of the first end of the second blood line 34, for allowing a flow of blood from the first blood line 33 to the eighth blood line 39 via the first conduit 61. This means that the inlet 51 of the second blood pump 50 is in fluid communication with the inlet section 11' of the blood withdrawal line 11, which corresponds to the control point 171 being open.

Simultaneously, the second conduit 62 faces the second end 382 of the seventh blood line 38 instead of the second end 372 of the third blood line 37, for allowing a flow of blood from the seventh blood line 38 to the fourth blood line 36 via the second conduit 62. This means that the outlet 42 of the first blood pump 40 is in fluid communication with the blood treatment unit 20, which corresponds to the control point 161 being open. Moreover, the third conduit 63 still faces the second end 352 of the fifth blood line 35 and faces the first end 321 of the sixth blood line 32.

As shown in Fig. 5a, in the second phase of double-needle treatment DN-P2 the first end 341 of the second blood line 34 and the second end 372 of the third blood line 37 are blocked by the peripheral boundary of the valve body 60. This means that no blood may enter the inlet 41 of the first blood pump 40 or may exit from the outlet 52 of the second blood pump 50, which corresponds to the control points 151 and 181 being closed.

Moreover, in the second phase of double-needle treatment DN-P2 the first control pump 106 feeds treatment fluid to the fluid chamber of the first blood pump 40, such that blood leaves the blood chamber of the first blood pump 40. At the same time the second control pump 105 draws treatment fluid from the fluid chamber of the second blood pump 50, such that blood fills the blood chamber of the second blood pump 50. Hence, by virtue of the control pumps and the second position of the valve, a flow of blood in the blood treatment unit 20 is generated as illustrated by Figs 5a and 5b, i.e. the second blood pump 50 draws blood from the blood source S and the first blood pump 40 feeds blood to the blood treatment unit 20 and further to the target vessel T.

During a continuous operation in a double-needle mode the valve is cyclically switched from the first position to the second position (DN-P1 to DN-P2) while the direction of the flows generated by the control pumps 105, 106 are changed in a corresponding, cyclic manner. The opening and closing at the control points and the asynchronous operation of the blood pumps render a continuous flow of blood from the blood source, through the blood treatment unit and to the target vessel, which is quite advantageous from an efficiency point of view.

For purpose of obtaining an even more smooth operation and flow of blood to and from a patient or to and from another type of blood source and target vessel, pressure meters and/or flow meters may be arranged in fluid lines and blood lines of the apparatus or blood treatment device. Such meters may send measured pressure- and flow values to the control unit which in turn may control the cyclic switching in dependence thereof.

As shown in Figs 5a and 5b, during double-needle treatment each of the fourth conduit 64, the fifth conduit 65, the sixth conduit 66 and the seventh conduit 67 faces the surface of the valve seat 70, such that these conduits are blocked from any blood flow.

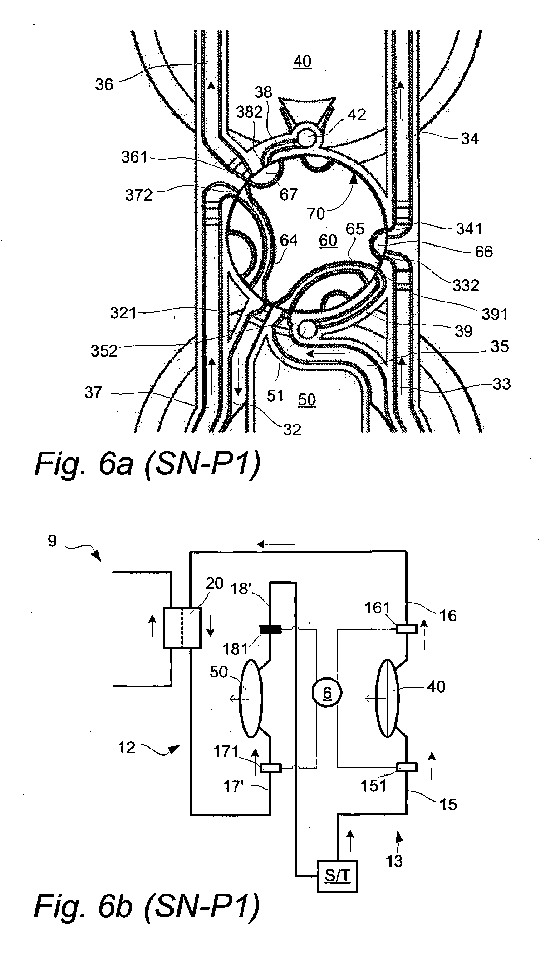

With reference to Figs 6a and 6b, operation of the apparatus 2 in the first phase of single-needle treatment (SN-P1) is illustrated. Since single-needle operation uses a common blood source and target vessel S/T, and hence the inlet section 11' of the blood withdrawal line 11 and the outlet section 12' of the blood return line 12 are connected to the common blood source and target vessel S/ T, for example via the Y-connection piece 19 illustrated in Fig. 1 and schematically shown by Fig. 6b.

In the first phase of single-needle treatment SN-P1 the control unit 107 sets the valve body 60 to the third position, such that the sixth conduit 66 faces the second end 332 of the first blood line 33 and faces the first end 341 of the second blood line 34, for allowing a flow of blood from the first blood line 33 to the second blood line 34 via the sixth conduit 66. This means that the blood source S is in fluid communication with the inlet of the first blood pump 40, which corresponds to the control point 151 being open.

At the same time the seventh conduit 67 faces the second end 382 of the seventh blood line 38 and faces the first end 361 of the fourth blood line 36, for allowing a flow of blood from the seventh blood line 38 to the fourth blood line,36. This means that the outlet 42 of the first blood pump 40 is in fluid communication with the inlet 21 of the blood treatment unit 20 via the seventh conduit 67, which corresponds to the control point 161 being open.

Simultaneously the fifth conduit 65 faces the second end 352 of the fifth blood line 35 and faces the first end of the eighth blood line 39, for allowing a flow of blood from the fifth blood line 35 to the eighth blood line 39 via the fifth conduit 65. This means that the inlet 51 of the second blood pump 50 is in fluid communication with the outlet 22 of the blood treatment unit 20 via the fifth conduit 65, which corresponds to the control point 171 being open.

Also, the fourth conduit 64 faces second end 372 of the third blood line 37 but does not face the first end 321 of the sixth blood line 32. This means that any flow from the outlet 53 of the second blood pump 50 to the downstream section 12' of the blood return line 12 is prevented, which corresponds to the control point 181 being closed.

Also, when the valve 6 is in the third position (and in the fourth position described below), the first conduit 61, the second conduit 62 and the third conduit 63 faces a wall of the valve seat 70, which prevents any flow of blood through the conduits 61, 62 and 63.

From this follows that, during the first phase of single-needle operation SN-P1, the pumps are connected in series with the first blood pump 40 upstream the blood treatment unit 20, i.e. in the blood withdrawal line 11, and with the second blood pump 50 downstream the blood treatment unit 20, i.e. in the blood return line 12 which is hence divided into an upstream section 17' and a downstream section 18'. Furthermore, the control pumps 105, 106 are in single-needle operation operated synchronously in the sense that each of the control pumps 105, 106 simultaneously feeds and draws treatment fluid to/from the fluid chambers of the blood pumps 40, 50, (i.e. the chambers of the blood pumps are filled respectively emptied simultaneously). More specifically, in the first phase of single-needle operation SN-P1 the control pumps 105, 106 simultaneously draws treatment fluid from the fluid chambers of the blood pumps 40, 50, such that the first blood pump 40 draws blood from the combined blood source and target vessel S/T while the second blood pump 50 draws blood from the blood treatment unit 20.

When relating the blood lines of Fig. 5a to the flow lines of Fig. 5b, it may in functional terms be said that the second blood line 34 in combination with the first blood line 33 correspond to the upstream section 15 while the seventh blood line 38 in combination with the fourth blood line 36 correspond to the downstream section 16. In a similar manner the fifth blood line 35 in combination with the eighth blood line 39 correspond to section 17', while the third blood line 37 in combination with the sixth blood line 32 correspond to section 18'.

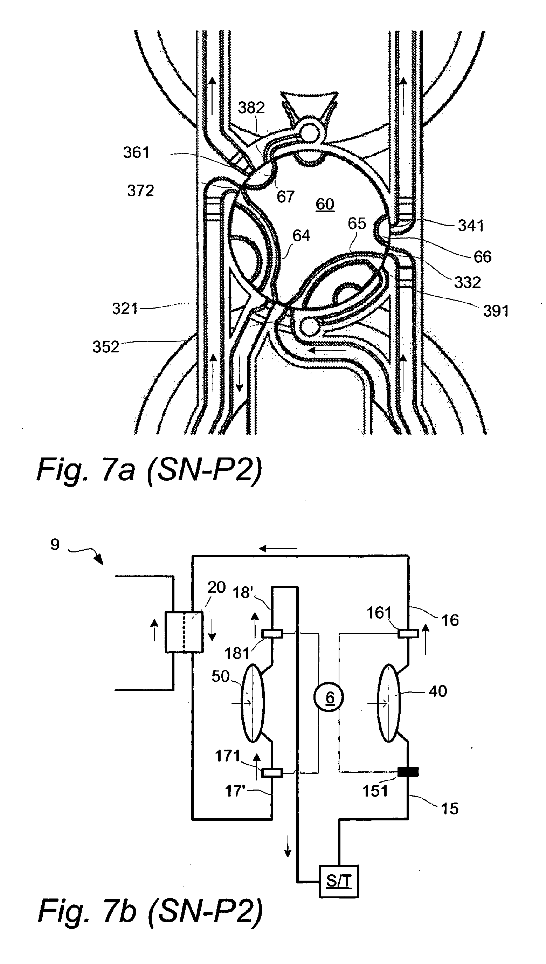

With reference to Figs 7a and 7b, operation of the apparatus 2 in the second phase of single-needle treatment (SN-P2) is illustrated. In this phase the control unit 107 sets the valve body 60 in the fourth position which corresponds to the third position illustrated by Figs 6a and valve 6, but with the differences that i) the sixth conduit 66 does not longer face the second end 332 of the sixth blood line 32, which corresponds to the control point 151 being closed, and ii) the fourth conduit 64 now faces the first end 321 of the sixth blood line 32, which corresponds to the control point 181 being open.

Moreover, in the second phase of single-needle operation SN-P2, each of the control pumps 105, 106 simultaneously feed treatment fluid to the respective fluid chamber of the blood pumps 40, 50, such that the first blood pump 40 feeds blood to the blood treatment unit 20 while the second blood pump 50 feeds blood to the combined blood source and target vessel S/T.

The described blood treatment device 10 and valve 6 may, as described above, be configured to perform double-needle or single-needle dialysis as selected by an operator that sets the control unit 107 such that it controls the valve 6 for movement between the positions previously described. However, according to another embodiment, the valve body 60 only comprises conduits for double-needle operation but none or not all conduits used for single-needle operation, i.e. the valve 6 comprises the first conduit 61, the second conduit 62 and the third conduit 63, but not any or some of the fourth conduit 64, the fifth conduit 65, the sixth conduit 66 and/or the seventh conduit 67. In a corresponding manner, according to a further embodiment, the valve body 60 only comprises conduits for single-needle operation but none or not all conduits used for double-needle operation. Still, for each embodiment the valve seat 70 comprises at least the six opening 71-72 or comprises all of the eight opening 71-78. This facilitates a blood treatment unit that may be readily manufactured to operate in double-needle operation, single-needle operation, or double- or single needle operation as selected by an operator, by selecting a proper valve body 60 during the manufacturing process. (Of course, the control unit 107 must before a dialysis operation be set to control the valve 6 accordingly.) Thus, the type of valve body 60 may define the type of available dialysis operation.

In this description, the wording that "a pump is arranged in a conduit/line" shall be understood to encompass all arrangements wherein the pump is configured to operate on a fluid passing through the conduit/line. I.e. the pump in question need not actually be included in the conduit. For example, the pump may be a hose pump which is configured to manipulate the exterior of a fluid conduit/line.

The components of the blood treatment unit 20 may advantageously be manufactured from material known within the field of blood treatment units. Also, the control per se of the pumps 104-106 and the actuator 7 may be implemented by conventional control methods within the field of blood treatment apparatuses.

Moreover, the various blood lines of the blood treatment unit 20 as well as conduits of the valve body 60 may be arranged in other ways than depicted, as long as a flow of blood through at least two blood lines may be controlled by moving the valve 6 (or valve body 60) between at least two different positions. Also, as the skilled person realizes, the control unit may comprise one or more data processors which each performs one or more of the described control operations. Thus, although various embodiments of the invention have been described and shown, the invention is not restricted thereto, but may also be embodied in other ways within the scope of the subject-matter defined by the appended claims.

## Claims

1. A blood treatment device comprising:
a blood treatment unit (20) having an inlet (21) and an outlet (22),
a blood withdrawal line (11) connected to the inlet (21) of the blood treatment unit (20), the blood withdrawal line (11) having a first branch line (13) and a second branch line (14) connected in parallel with the first branch line (13),
a blood return line (12) connected to the outlet (22) of the blood treatment unit (20),
a first pump (40) connected in the first branch line (13) such that the first branch line (13) is divided into an upstream section (15) and a downstream section (16),
a second pump (50) connected in the second branch line (14) such that the second branch line (14) is divided into an upstream section (17) and a downstream section (18),
**characterized by**
a valve (6) configured to:
- open the upstream section (15) and close the downstream section (16) of the first branch line (13), and close the upstream section (17) and open the downstream section (18) of the second branch line (14), when the valve (6) is moved to a first position (DN-P1), and
- close the upstream section (15) and open the downstream section (16) of the first branch line (13), and open the upstream section (17) and close the downstream section (18) of the second branch line (14), when the valve (6) is moved to a second position (DN-P2).

2. A blood treatment device according to claim 1, wherein the valve (6) comprises a valve body (60) and a valve seat (70) in which the valve body (60) is arranged.

3. A blood treatment device according to claim 1 or 2, wherein the valve comprises a closure element (29) connected to the valve seat (70), such that the valve body (60) is enclosed by the valve seat (70) and the closure element (29).

4. A blood treatment device according to any one of claims 1 - 3, wherein the valve (6) is configured to move between the first position (DN-P1) and the second position (DN-P2) by rotation of the valve (6).

5. A blood treatment device according to any one of claims 1 - 4, wherein the valve (6) has a circular shape.

6. A blood treatment device according to any one of claims 1 - 5, wherein the valve (6) comprises an engagement member (68) configured to interact with an actuator (7) of a dialysis apparatus (2), for allowing the valve (6) to be moved between the first position (DN-P1) and the second position (DN-P2).

7. A blood treatment device according to any one of claims 1 - 6, wherein the valve (6) comprises:
a first conduit (61) configured to provide the opening and closing of the upstream sections (15, 17) of the first and second branch lines (13, 14), and
a second conduit (62) configured to provide the opening and closing of the downstream sections (16, 18) of the first and second branch lines (13, 14).

8. A blood treatment device according to claim 7, wherein the valve (6) comprises a third conduit (63) arranged in the blood return line (12).

9. A blood treatment device according to claim 2 and 7 or 8, wherein the valve body (60) comprises the first (61) and second (62) conduits.

10. A blood treatment device according to any one of claims 1 - 9, wherein the valve (6) comprises at least six openings (71-76), wherein each of a blood inlet (331) of the blood treatment device, pump inlets (41,51) and pump outlets (42, 52) of the pumps (40, 50), and the inlet (21) of the blood treatment unit (20) are connected to a respective opening of the openings (71-76).

11. A blood treatment device according to claim 10, wherein the valve (6) comprises two further openings (77, 78), wherein each of the outlet (22) of the blood treatment unit (20) and a blood outlet (322) of the blood treatment device are connected to a respective opening of the further openings (77-78).

12. A blood treatment device according to claim 2 and 10 or 11, wherein the valve seat (70) comprises the six openings (71-76).

13. A blood treatment device according to any one of claims 1 - 12, wherein the valve (6) is configured to:
disconnect the second branch line (14) from the first branch line (13) and connect the second pump (50) in the blood return line (12), such that the blood return line (12) is divided into an upstream section (17') and a downstream section (18'), when the valve (6) is in any of a third position (SN-P1) and a fourth position (SN-P2),
open the upstream section (15) of the first branch line (13) and close the downstream section (18') of the blood return line (12), when the valve (6) is moved to the third position (SN-P1), and
close the upstream section (15) of the first branch line (13) and open the downstream section (18') of the blood return line (12), when the valve (6) is moved to the fourth position (SN-P2).

14. A blood treatment device according to any one of claims 1 - 13, wherein the valve (6) comprises a conduit (64) configured to provide opening and closing of the downstream section (18') of the blood return line (12).

15. A blood treatment device according to any one of claims 1 - 14, wherein the blood treatment unit (20) has an elongated shape, the valve (6) being arranged at a long side (9) of the blood treatment unit (20).

16. A blood treatment device according to any one of claims 1 - 15, wherein each of the first pump (40) and the second pump (50) are arranged at a long side (9) of the blood treatment unit (20).

17. A blood treatment device according to any one of claims 1 - 16, wherein each of the first pump (40) and the second pump (50) are membrane pumps.

18. A blood treatment device comprising:
a blood treatment unit (20) having an inlet (21) and an outlet (22),
a blood withdrawal line (11) connected to the inlet (21) of the blood treatment unit (20),
a blood return line (12) connected to the outlet (22) of the blood treatment unit (20),
a first pump (40) connected in the blood withdrawal line (11) such that the blood withdrawal line (11) is divided into an upstream section (15) and a downstream section (16),
a second pump (50) connected in the blood return line (12) such that the blood return line (12) is divided into an upstream section (17') and a downstream section (18'),
**characterized by**
a valve (6) configured to:
- open the upstream section (15) of the blood withdrawal line (11) and close the downstream section (18') of the blood return line (12), when the valve (6) is moved to a third position (SN-P1), and
- close the upstream section (15) of the blood withdrawal line (11) and open the downstream section (18') of the blood return line (12), when the valve (6) is moved to a fourth position (SN-P2).

19. A blood treatment device according to claim 18, wherein the valve (6) comprises a valve body (60) and a valve seat (70) in which the valve body (60) is arranged.

20. A blood treatment device according to claim 18 or 19, wherein the valve comprises a closure element (29) connected to the valve seat (70), such that the valve body (60) is enclosed by the valve seat (70) and the closure element (29).

21. A blood treatment device according to any one of claims 18 - 20, wherein the valve (6) is configured to move between the third position (SN-P1) and the fourth position (SN-P2) by rotation of the valve (6).

22. A blood treatment device according to any one of claims 18 - 21, wherein the valve (6) has a circular shape.

23. A blood treatment device according to any one of claims 18 - 22, wherein the valve (6) comprises an engagement member (68) configured to interact with an actuator (7) of a dialysis apparatus (2), for allowing the valve (6) to be moved between the third position (SN-P1) and the fourth position (SN-P2).

24. A blood treatment device according to any one of claims 18 - 23, wherein the valve (6) comprises:
a sixth conduit (66) configured to provide the opening and closing of the upstream section (15) of the blood withdrawal line (11), and
a fourth conduit (64) configured to provide the opening and closing of the downstream section (18') of the blood return line (12).

25. A blood treatment device according to claim 24, wherein the valve (6) comprises a fifth conduit (65) and a seventh conduit (67) respectively arranged downstream the sixth conduit (66) and upstream the fourth conduit (64).

26. A blood treatment device according to claim 19 and 24 or 25, wherein the valve body (60) comprises the sixth (66) and fourth (64) conduits.

27. A blood treatment device according to any one of claims 18 - 26, wherein the valve (6) comprises at least six openings (71-76), wherein each of a blood inlet (331) of the blood treatment device, pump inlets (41, 51) and pump outlets (42, 52) of the pumps (40, 50), and the inlet (21) of the blood treatment unit (20) are connected to a respective opening of the openings (71-76).

28. A blood treatment device according to claim 27, wherein the valve (6) comprises two further openings (77, 78), wherein each of the outlet (22) of the blood treatment unit (20) and a blood outlet (322) of the blood treatment device are connected to a respective opening of the further openings (77-78).

29. A blood treatment device according to claim 19 and 27 or 28, wherein the valve seat (70) comprises the six openings (71-76).

30. A blood treatment device according to any one of claims 18 - 29, wherein the valve (6) is configured to:
disconnect the second pump (50) from the blood return line (12), divide the blood withdrawal line (11) into a first branch line (13) that includes the first blood pump (40) and into a second branch line (14) connected in parallel with the first branch line (13), and connect the second blood pump (50) in the second branch line (14), when the valve (6) is in any of a first position (DN-P1) and a second position (DN-P2),
open an upstream section (15) and close a downstream section (16) of the first branch line (13), and close an upstream section (17) and open a downstream section (18) of the second branch line (14), when the valve (6) is moved to the first position (DN-P1), and
close the upstream section (15) and open the downstream section (16) of the first branch line (13), and open the upstream section (17) and close the downstream section (18) of the second branch line (14), when the valve (6) is moved to the second position (DN-P2).

31. A blood treatment device according to any one of claims 18 - 30, wherein the valve (6) comprises a conduit (63) configured to disconnect the second pump (50) from the blood return line (12).

32. A blood treatment device according to any one of claims 18 - 31, wherein the blood treatment unit (20) has an elongated shape, the valve (6) being arranged at a long side (9) of the blood treatment unit (20).

33. A blood treatment device according to any one of claims 18 - 32 wherein each of the first pump (40) and the second pump (50) are arranged at a long side (9) of the blood treatment unit (20).

34. A blood treatment device according to any one of claims 18 - 33, wherein each of the first pump (40) and the second pump (50) are membrane pumps.

35. A blood treatment device comprising:
a blood treatment unit (20) having an inlet (21) and an outlet (22) a blood withdrawal line (33) configured to receive blood a blood return line (32) configured to return blood,
a first pump (40) having an inlet (41) and an outlet (42),
a second pump (50) having an inlet (51) and an outlet (52),
**characterized by**
a valve seat (70) comprising at least six openings (71-76), wherein each of the inlet (21) of the blood treatment unit (20), the blood withdrawal line (33), the inlet (41) and outlet (42) of the first pump (40), and the inlet (51) and the outlet (52) of the second pump (50) are connected to a respective opening of the six openings (71-76), for allowing a valve body (60) arranged in the valve seat (70) to control a flow of blood via at least two of the six openings (71-76).

36. A blood treatment device according to claim 35, wherein the valve seat (70) comprises two further openings (77, 78), wherein each of the blood return line (32) and the outlet (22) of the blood treatment unit (20) are connected to a respective opening of the further openings (77-78).

37. A blood treatment apparatus comprising a blood treatment device (10) according to any one of claims 1 - 36.

## Patentansprüche

1. Blutbehandlungsvorrichtung, umfassend:
eine Blutbehandlungseinheit (20) mit einem Einlass (21) und einem Auslass (22),
eine Blutentnahmeleitung (11), die mit dem Einlass (21) der Blutbehandlungseinheit (20) verbunden ist, wobei die Blutentnahmeleitung (11) eine erste Abzweigungsleitung (13) und ein zweite Abzweigungsleitung (14), die parallel zu der ersten Abzweigungsleitung (13) angeordnet ist, aufweist,
eine Blutrückflussleitung (12), die mit dem Auslass (22) der Blutbehandlungseinheit (20) verbunden ist,
eine erste Pumpe (40), die in der ersten Abzweigungsleitung (13) derart angeschlossen ist, dass die erste Abzweigungsleitung (13) in einen Abschnitt strömungsaufwärts (15) und einen Abschnitt strömungsabwärts (16) unterteilt ist,
eine zweite Pumpe (50), die in der zweiten Abzweigungsleitung (14) derart angeschlossen ist, dass die zweite Abzweigungsleitung (14) in einen Abschnitt strömungsaufwärts (17) und einen Abschnitt strömungsabwärts (18) unterteilt ist,
**gekennzeichnet durch**
ein Ventil (6), das so eingerichtet ist, dass es:
- den Abschnitt strömungsaufwärts (15) der ersten Abzweigungsleitung (13) öffnet und ihren Abschnitt strömungsabwärts (16) schließt, und den Abschnitt strömungsaufwärts (17) der zweiten Abzweigungsleitung (14) schließt und ihren Abschnitt strömungsabwärts (18) öffnet, wenn das Ventil (6) in eine erste Stellung (DN-P1) bewegt wird, und
- den Abschnitt strömungsaufwärts (15) der ersten Abzweigungsleitung (13) schließt und ihren Abschnitt strömungsabwärts (16) öffnet, und den Abschnitt strömungsaufwärts (17) der zweiten Abzweigungsleitung (14) öffnet und ihren Abschnitt strömungsabwärts (18) schließt, wenn das Ventil (6) in eine zweite Stellung (DN-P2) bewegt wird.

2. Blutbehandlungsvorrichtung nach Anspruch 1, wobei das Ventil (6) einen Ventilkörper (60) und einen Ventilsitz (70) umfasst, in dem der Ventilkörper (60) angeordnet ist.

3. Blutbehandlungsvorrichtung nach Anspruch 1 oder 2, wobei das Ventil ein Abschlusselement (29) umfasst, das mit dem Ventilsitz (70) derart verbunden ist, dass der Ventilkörper (60) von dem Ventilsitz (70) und dem Abschlusselement (29) umgeben ist.

4. Blutbehandlungsvorrichtung nach einem der Ansprüche 1 bis 3, wobei das Ventil (6) so eingerichtet ist, dass es sich zwischen der ersten Stellung (DN-P1) und der zweiten Stellung (DN-P2) durch Drehung des Ventils (6) bewegt.

5. Blutbehandlungsvorrichtung nach einem der Ansprüche 1 bis 4, wobei das Ventil (6) eine Kreisform aufweist.

6. Blutbehandlungsvorrichtung nach einem der Ansprüche 1 bis 5, wobei das Ventil (6) ein Eingriffelement (68) umfasst, das so eingerichtet ist, dass es mit einem Stellglied (7) eines Dialysegeräts (2) in Wechselwirkung steht, um zu ermöglichen, dass das Ventil (6) zwischen der ersten Stellung (DN-P1) und der zweiten Stellung (DN-P2) bewegt wird.

7. Blutbehandlungsvorrichtung nach einem der Ansprüche 1 bis 6, wobei das Ventil (6) Folgendes umfasst:
einen ersten Durchlass (61), der so eingerichtet ist, dass er für das Öffnen und Schließen der Abschnitte strömungsaufwärts (15, 17) der ersten und zweiten Abzweigungsleitung (13, 14) sorgt, und
einen zweiten Durchlass (62), der so eingerichtet ist, dass er für das Öffnen und Schließen der Abschnitte strömungsabwärts (16, 18) der ersten und zweiten Abzweigungsleitung (13, 14) sorgt.

8. Blutbehandlungsvorrichtung nach Anspruch 7, wobei das Ventil (6) einen dritten Durchlass (63) umfasst, der in der Blutrückflussleitung (12) angeordnet ist.

9. Blutbehandlungsvorrichtung nach Anspruch 2 und 7 oder 8, wobei der Ventilkörper (60) den ersten (61) und zweiten (62) Durchlass umfasst.

10. Blutbehandlungsvorrichtung nach einem der Ansprüche 1 bis 9, wobei das Ventil (6) mindestens sechs Öffnungen (71 bis 76) umfasst, wobei ein Bluteinlass (331) der Blutbehandlungsvorrichtung, Pumpeneinlässe (41, 51) und Pumpenauslässe (42, 52) der Pumpen (40, 50) und der Einlass (21) der Blutbehandlungseinheit (20) jeweils mit einer jeweiligen Öffnung der Öffnungen (71 bis 76) verbunden sind.

11. Blutbehandlungsvorrichtung nach Anspruch 10, wobei das Ventil (6) zwei weitere Öffnungen (77, 78) umfasst, wobei der Auslass (22) der Blutbehandlungseinheit (20) und ein Blutauslass (322) der Blutbehandlungsvorrichtung jeweils mit einer jeweiligen Öffnung der weiteren Öffnungen (77 und 78) verbunden sind.

12. Blutbehandlungsvorrichtung nach Anspruch 2 und 10 oder 11, wobei der Ventilsitz (70) die sechs Öffnungen (71 bis 76) umfasst.

13. Blutbehandlungsvorrichtung nach einem der Ansprüche 1 bis 12, wobei das Ventil (6) so eingerichtet ist, dass es:
die zweite Abzweigungsleitung (14) von der ersten Abzweigungsleitung (13) trennt und die zweite Pumpe (50) in der Blutrückflussleitung (12) anbindet, sodass die Blutrückflussleitung (12) in einen Abschnitt strömungsaufwärts (17') und einen Abschnitt strömungsabwärts (18') unterteilt ist, wenn sich das Ventil (6) in einer dritten Stellung (SN-P1) oder einer vierten Stellung (SN-P2) befindet,
den Abschnitt strömungsaufwärts (15) der ersten Abzweigungsleitung (13) öffnet und den Abschnitt strömungsabwärts (18') der Blutrückflussleitung (12) schließt, wenn das Ventil (6) in die dritte Stellung (SN-P1) bewegt wird, und
den Abschnitt strömungsaufwärts (15) der ersten Abzweigungsleitung (13) schließt und den Abschnitt strömungsabwärts (18') der Blutrückflussleitung (12) öffnet, wenn das Ventil (6) in die vierte Stellung (SN-P2) bewegt wird.

14. Blutbehandlungsvorrichtung nach einem der Ansprüche 1 bis 13, wobei das Ventil (6) einen Durchlass (64) umfasst, der so eingerichtet ist, dass er für das Öffnen und Schließen des Abschnitts strömungsabwärts (18') der Blutrückflussleitung (12) sorgt.

15. Blutbehandlungsvorrichtung nach einem der Ansprüche 1 bis 14, wobei die Blutbehandlungseinheit (20) eine längliche Form aufweist, wobei das Ventil (6) an einer langen Seite (9) der Blutbehandlungseinheit (20) angeordnet ist.

16. Blutbehandlungsvorrichtung nach einem der Ansprüche 1 bis 15, wobei die erste Pumpe (40) und die zweite Pumpe (50) jeweils an einer langen Seite (9) der Blutbehandlungseinheit (20) angeordnet sind.

17. Blutbehandlungsvorrichtung nach einem der Ansprüche 1 bis 16, wobei die erste Pumpe (40) und die zweite Pumpe (50) jeweils Membranpumpen sind.

18. Blutbehandlungsvorrichtung, umfassend:
eine Blutbehandlungseinheit (20) mit einem Einlass (21) und einem Auslass (22),
eine Blutentnahmeleitung (11), die mit dem Einlass (21) der Blutbehandlungseinheit (20) verbunden ist,
eine Blutrückflussleitung (12), die mit dem Auslass (22) der Blutbehandlungseinheit (20) verbunden ist,
eine erste Pumpe (40), die in der Blutentnahmeleitung (11) derart angeschlossen ist, dass die Blutentnahmeleitung (11) in einen Abschnitt strömungsaufwärts (15) und einen Abschnitt strömungsabwärts (16) unterteilt ist,
eine zweite Pumpe (50), die in der Blutrückflussleitung (12) derart angeschlossen ist, dass die Blutrückflussleitung (12) in einen Abschnitt strömungsaufwärts (17') und einen Abschnitt strömungsabwärts (18') unterteilt ist, **gekennzeichnet durch**
ein Ventil (6), das so eingerichtet ist, dass es:
- den Abschnitt strömungsaufwärts (15) der Blutentnahmeleitung (11) öffnet und den Abschnitt strömungsabwärts (18') der Blutrückflussleitung (12) schließt, wenn das Ventil (6) in eine dritte Stellung (SN-P1) bewegt wird, und
- den Abschnitt strömungsaufwärts (15) der Blutentnahmeleitung (11) schließt und den Abschnitt strömungsabwärts (18') der Blutrückflussleitung (12) öffnet, wenn das Ventil (6) in eine vierte Stellung (SN-P2) bewegt wird.

19. Blutbehandlungsvorrichtung nach Anspruch 18, wobei das Ventil (6) einen Ventilkörper (60) und einen Ventilsitz (70) umfasst, in dem der Ventilkörper (60) angeordnet ist.

20. Blutbehandlungsvorrichtung nach Anspruch 18 oder 19, wobei das Ventil ein Abschlusselement (29) umfasst, das mit dem Ventilsitz (70) derart verbunden ist, dass der Ventilkörper (60) von dem Ventilsitz (70) und dem Abschlusselement (29) umgeben ist.

21. Blutbehandlungsvorrichtung nach einem der Ansprüche 18 bis 20, wobei das Ventil (6) so eingerichtet ist, dass es sich zwischen der dritten Stellung (SN-P1) und der vierten Stellung (SN-P2) durch Drehung des Ventils (6) bewegt.

22. Blutbehandlungsvorrichtung nach einem der Ansprüche 18 bis 21, wobei das Ventil (6) eine Kreisform aufweist.

23. Blutbehandlungsvorrichtung nach einem der Ansprüche 18 bis 22, wobei das Ventil (6) ein Eingriffelement (68) umfasst, das so eingerichtet ist, dass es mit einem Stellglied (7) eines Dialysegeräts (2) in Wechselwirkung steht, um zu ermöglichen, dass das Ventil (6) zwischen der dritten Stellung (SN-P1) und der vierten Stellung (SN-P2) bewegt wird.

24. Blutbehandlungsvorrichtung nach einem der Ansprüche 18 bis 23, wobei das Ventil (6) Folgendes umfasst:
einen sechsten Durchlass (66), der so eingerichtet ist, dass er für das Öffnen und Schließen des Abschnitts strömungsaufwärts (15) der Blutentnahmeleitung (11) sorgt, und
einen vierten Durchlass (64), der so eingerichtet ist, dass er für das Öffnen und Schließen des Abschnitts strömungsabwärts (18') der Blutrückflussleitung (12) sorgt.

25. Blutbehandlungsvorrichtung nach Anspruch 24, wobei das Ventil (6) einen fünften Durchlass (65) und einen siebenten Durchlass (67) umfasst, der strömungsabwärts des sechsten Durchlasses (66) beziehungsweise strömungsaufwärts des vierten Durchlasses (64) angeordnet ist.

26. Blutbehandlungsvorrichtung nach Anspruch 19 und 24 oder 25, wobei der Ventilkörper (60) den sechsten (66) und vierten (64) Durchlass umfasst.

27. Blutbehandlungsvorrichtung nach einem der Ansprüche 18 bis 26, wobei das Ventil (6) mindestens sechs Öffnungen (71 bis 76) umfasst, wobei ein Bluteinlass (331) der Blutbehandlungsvorrichtung, Pumpeneinlässe (41, 51) und Pumpenauslässe (42, 52) der Pumpen (40, 50) sowie der Einlass (21) der Blutbehandlungseinheit (20) jeweils mit einer jeweiligen Öffnung der Öffnungen (71 bis 76) verbunden sind.

28. Blutbehandlungsvorrichtung nach Anspruch 27, wobei das Ventil (6) zwei weitere Öffnungen (77, 78) umfasst, wobei der Auslass (22) der Blutbehandlungseinheit (20) und ein Blutauslass (322) der Blutbehandlungsvorrichtung jeweils mit einer jeweiligen Öffnung der weiteren Öffnungen (77 und 78) verbunden sind.

29. Blutbehandlungsvorrichtung nach Anspruch 19 und 27 oder 28, wobei der Ventilsitz (70) die sechs Öffnungen (71 bis 76) umfasst.

30. Blutbehandlungsvorrichtung nach einem der Ansprüche 18 bis 29, wobei das Ventil (6) so eingerichtet ist, dass es:
die zweite Pumpe (50) von der Blutrückflussleitung (12) trennt, die Blutentnahmeleitung (11) in eine erste Abzweigungsleitung (13), die die erste Blutpumpe (40) aufweist, und in eine zweite Abzweigungsleitung (14), die parallel zu der ersten Abzweigungsleitung (13) angeordnet ist, aufteilt, und die zweite Blutpumpe (50) in der zweiten Abzweigungsleitung (14) anbindet, wenn sich das Ventil (6) in einer ersten Stellung (DN-P1) oder einer zweiten Stellung (DN-P2) befindet,
einen Abschnitt strömungsaufwärts (15) der ersten Abzweigungsleitung (13) öffnet und einen Abschnitt strömungsabwärts (16) davon schließt, und einen Abschnitt strömungsaufwärts (17) der zweiten Abzweigungsleitung (14) schließt und einen Abschnitt strömungsabwärts (18) davon öffnet, wenn das Ventil (6) in die erste Stellung (DN-P1) bewegt wird, und
den Abschnitt strömungsaufwärts (15) der ersten Abzweigungsleitung (13) schließt und ihren Abschnitt strömungsabwärts (16) öffnet, und den Abschnitt strömungsaufwärts (17) der zweiten Abzweigungsleitung (14) öffnet und ihren Abschnitt strömungsabwärts (18) schließt, wenn das Ventil (6) in die zweite Stellung (DN-P2) bewegt wird.

31. Blutbehandlungsvorrichtung nach einem der Ansprüche 18 bis 30, wobei das Ventil (6) einen Durchlass (63) umfasst, der so eingerichtet ist, dass er die zweite Pumpe (50) von der Blutrückflussleitung (12) trennt.

32. Blutbehandlungsvorrichtung nach einem der Ansprüche 18 bis 31, wobei die Blutbehandlungseinheit (20) eine längliche Form aufweist, wobei das Ventil (6) an einer langen Seite (9) der Blutbehandlungseinheit (20) angeordnet ist.

33. Blutbehandlungsvorrichtung nach einem der Ansprüche 18 bis 32, wobei die erste Pumpe (40) und die zweite Pumpe (50) jeweils an einer langen Seite (9) der Blutbehandlungseinheit (20) angeordnet sind.

34. Blutbehandlungsvorrichtung nach einem der Ansprüche 18 bis 33, wobei die erste Pumpe (40) und die zweite Pumpe (50) jeweils Membranpumpen sind.

35. Blutbehandlungsvorrichtung, umfassend:
eine Blutbehandlungseinheit (20) mit einem Einlass (21) und einem Auslass (22)
eine Blutentnahmeleitung (33), die so eingerichtet ist, dass sie Blut aufnimmt
eine Blutrückflussleitung (32), die so eingerichtet ist, dass sie Blut zurückleitet,
eine erste Pumpe (40) mit einem Einlass (41) und einem Auslass (42),
eine zweite Pumpe (50) mit einem Einlass (51) und einem Auslass (52),
**gekennzeichnet durch**
einen Ventilsitz (70), der mindestens sechs Öffnungen (71 bis 76) umfasst, wobei der Einlass (21) der Blutbehandlungseinheit (20), die Blutentnahmeleitung (33), der Einlass (41) und Auslass (42) der ersten Pumpe (40) und der Einlass (51) und der Auslass (52) der zweiten Pumpe (50) jeweils mit einer jeweiligen Öffnung der sechs Öffnungen (71 bis 76) verbunden sind, um zu ermöglichen, dass ein Ventilkörper (60), der in dem Ventilsitz (70) angeordnet ist, einen Blutstrom über mindestens zwei der sechs Öffnungen (71 bis 76) steuert.

36. Blutbehandlungsvorrichtung nach Anspruch 35, wobei der Ventilsitz (70) zwei weitere Öffnungen (77, 78) umfasst, wobei die Blutrückflussleitung (32) und der Auslass (22) der Blutbehandlungseinheit (20) jeweils mit einer jeweiligen Öffnung der weiteren Öffnungen (77 und 78) verbunden sind.

37. Blutbehandlungsgerät, das eine Blutbehandlungsvorrichtung (10) nach einem der Ansprüche 1 bis 36 umfasst.

## Revendications

1. Dispositif pour le traitement du sang, comprenant :
une unité de traitement du sang (20) comportant une entrée (21) et une sortie (22),
une ligne de retrait du sang (11) raccordée à l'entrée (21) de l'unité de traitement du sang (20), la ligne de retrait du sang (11) comportant une première ligne d'embranchement (13) et une seconde ligne d'embranchement (14) raccordée en parallèle avec la première ligne d'embranchement (13),
une ligne de retour du sang (12) raccordée à la sortie (22) de l'unité de traitement du sang (20),
une première pompe (40) raccordée dans la première ligne d'embranchement (13) de sorte que la première ligne d'embranchement (13) est divisée en une partie amont (15) et une partie aval (16),
une seconde pompe (50) raccordée dans la seconde ligne d'embranchement (14) de sorte que la seconde ligne d'embranchement (14) est divisée en une partie amont (17) et une partie aval (18),
**caractérisé par**
une valve (6) configurée pour :
- ouvrir la partie amont (15) et fermer la partie aval (16) de la première ligne d'embranchement (13), et fermer la partie amont (17) et ouvrir la partie aval (18) de la seconde ligne d'embranchement (14) quand la valve (6) est déplacée dans une première position (DN-P1), et
- fermer la partie amont (15) et ouvrir la partie aval (16) de la première ligne d'embranchement (13), et ouvrir la partie amont (17) et fermer la partie aval (18) de la seconde ligne d'embranchement (14) quand la valve (6) est déplacée dans une seconde position (DN-P2).

2. Dispositif pour le traitement du sang selon la revendication 1, la valve (6) comprenant un corps de valve (60) et un siège de valve (70) dans lequel le corps de valve (60) est disposé.

3. Dispositif pour le traitement du sang selon la revendication 1 ou 2, la valve comprenant un élément de fermeture (29) raccordé au siège de valve (70) de sorte que le corps de valve (60) est enfermé par le siège de valve (70) et l'élément de fermeture (29).

4. Dispositif pour le traitement du sang selon l'une quelconque des revendications 1 à 3, la valve (6) étant configurée pour se déplacer entre la première position (DN-P1) et la seconde position (DN-P2) par rotation de la valve (6).

5. Dispositif pour le traitement du sang selon l'une quelconque des revendications 1 à 4, la valve (6) présentant une forme circulaire.

6. Dispositif pour le traitement du sang selon l'une quelconque des revendications 1 à 5, la valve (6) comprenant un élément de prise (68) configuré pour agir réciproquement avec un actionneur (7) d'un appareil de dialyse (2), afin de permettre à la valve (6) d'être déplacée entre la première position (DN-P1) et la seconde position (DN-P2).

7. Dispositif pour le traitement du sang selon l'une quelconque des revendications 1 à 6, la valve (6) comprenant :
un premier conduit (61) configuré pour assurer l'ouverture et la fermeture des parties amont (15, 17) des première et seconde lignes d'embranchement (13, 14), et
un second conduit (62) configuré pour assurer l'ouverture et la fermeture des parties aval (16, 18) des première et seconde lignes d'embranchement (13, 14).

8. Dispositif pour le traitement du sang selon la revendication 7, la valve (6) comprenant un troisième conduit (63) disposé dans la ligne de retour du sang (12).

9. Dispositif de traitement du sang selon la revendication 2 et 7 ou 8, le corps de valve (60) comprenant les premier (61) et second (62) conduits.

10. Dispositif pour le traitement du sang selon l'une quelconque des revendications 1 à 9, la valve (6) comprenant au moins six ouvertures (71-76), une entrée du sang (331) du dispositif pour le traitement du sang, des entrées de pompe (41, 51) et des sorties de pompe (42, 52) des pompes (40, 50), et l'entrée (21) de l'unité de traitement du sang (20) étant raccordées chacune à une ouverture respective des ouvertures (71-76).

11. Dispositif pour le traitement du sang selon la revendication 10, la valve (6) comprenant deux autres ouvertures (77, 78), la sortie (22) de l'unité de traitement du sang (20) et une sortie du sang (322) du dispositif pour le traitement du sang étant raccordées chacune à une ouverture respective des autres ouvertures (77-78).

12. Dispositif pour le traitement du sang selon la revendication 2 et 10 ou 11, le siège de valve (70) comprenant les six ouvertures (71-76).

13. Dispositif pour le traitement du sang selon l'une quelconque des revendications 1 à 12, la valve (6) étant configurée pour :
séparer la seconde ligne d'embranchement (14) de la première ligne d'embranchement (13) et raccorder la seconde pompe (50) dans la ligne de retour du sang (12), de sorte que la ligne de retour du sang (12) est divisée en une partie amont (17') et une partie aval (18') quand la valve (6) est soit dans une troisième position (SN-P1) soit dans une quatrième position (SN-P2),
ouvrir la partie amont (15) de la première ligne d'embranchement (13) et fermer la partie aval (18') de la ligne de retour du sang (12) quand la valve (6) est déplacée dans la troisième position (SN-P1), et
fermer la partie amont (15) de la première ligne d'embranchement (13) et ouvrir la partie aval (18') de la ligne de retour du sang (12) quand la valve (6) est déplacée dans la quatrième position (SN-P2).

14. Dispositif pour le traitement du sang selon l'une quelconque des revendications 1 à 13, la valve (6) comprenant un conduit (64) configuré pour assurer l'ouverture et la fermeture de la partie aval (18') de la ligne de retour du sang (12).

15. Dispositif pour le traitement du sang selon l'une quelconque des revendications 1 à 14, l'unité de traitement du sang (20) présentant une forme allongée, la valve (6) étant disposée sur un côté long (9) de l'unité de traitement du sang (20).

16. Dispositif pour le traitement du sang selon l'une quelconque des revendications 1 à 15, la première pompe (40) et la seconde pompe (50) étant disposées chacune sur un côté long (9) de l'unité de traitement du sang (20).

17. Dispositif pour le traitement du sang selon l'une quelconque des revendications 1 à 16, la première pompe (40) et la seconde pompe (50) étant chacune une pompe à membrane.

18. Dispositif pour le traitement du sang, comprenant :
une unité de traitement du sang (20) comportant une entrée (21) et une sortie (22),
une ligne de retrait du sang (11) raccordée à l'entrée (21) de l'unité de traitement du sang (20),
une ligne de retour du sang (12) raccordée à la sortie (22) de l'unité de traitement du sang (20),
une première pompe (40) raccordée dans la ligne de retrait du sang (11) de sorte que la ligne de retrait du sang (11) est divisée en une partie amont (15) et une partie aval (16),
une seconde pompe (50) raccordée dans la ligne de retour du sang (12) de sorte que la ligne de retour du sang (12) est divisée en une partie amont (17') et une partie aval (18'),
**caractérisé par**
une valve (6) configurée pour :
- ouvrir la partie amont (15) de la ligne de retrait du sang (11) et fermer la partie aval (18') de la ligne de retour du sang (12) quand la valve (6) est déplacée dans une troisième position (SN-P1), et
- fermer la partie amont (15) de la ligne de retrait du sang (11) et ouvrir la partie aval (18') de la ligne de retour du sang (12) quand la valve (6) est déplacée dans une quatrième position (SN-P2).

19. Dispositif pour le traitement du sang selon la revendication 18, la valve (6) comprenant un corps de valve (60) et un siège de valve (70) dans lequel le corps de valve (60) est disposé.

20. Dispositif pour le traitement du sang selon la revendication 18 ou 19, la valve comprenant un élément de fermeture (29) raccordé au siège de valve (70) de sorte que le corps de valve (60) est enfermé par le siège de valve (70) et l'élément de fermeture (29).

21. Dispositif pour le traitement du sang selon l'une quelconque des revendications 18 à 20, la valve (6) étant configurée pour se déplacer entre la troisième position (SN-P1) et la quatrième position (SN-P2) par rotation de la valve (6).

22. Dispositif pour le traitement du sang selon l'une quelconque des revendications 18 à 21, la valve (6) présentant une forme circulaire.

23. Dispositif pour le traitement du sang selon l'une quelconque des revendications 18 à 22, la valve (6) comprenant un élément de prise (68) configuré pour agir réciproquement avec un actionneur (7) d'un appareil de dialyse (2), afin de permettre à la valve (6) d'être déplacée entre la troisième position (SN-P1) et la quatrième position (SN-P2).

24. Dispositif pour le traitement du sang selon l'une quelconque des revendications 18 à 23, la valve (6) comprenant :
un sixième conduit (66) configuré pour assurer l'ouverture et la fermeture de la partie amont (15) de la ligne de retrait du sang (11), et
un quatrième conduit (64) configuré pour assurer l'ouverture et la fermeture de la partie aval (18') de la ligne de retour du sang (12).

25. Dispositif pour le traitement du sang selon la revendication 24, la valve (6) comprenant un cinquième conduit (65) et un septième conduit (67) disposés respectivement en aval du sixième conduit (66) et en amont du quatrième conduit (64).

26. Dispositif de traitement du sang selon la revendication 19 et 24 ou 25, le corps de valve (60) comprenant les sixième (66) et quatrième (64) conduits.

27. Dispositif pour le traitement du sang selon l'une quelconque des revendications 18 à 26, la valve (6) comprenant au moins six ouvertures (71-76), une entrée du sang (331) du dispositif pour le traitement du sang, des entrées de pompe (41, 51) et des sorties de pompe (42, 52) des pompes (40, 50), et l'entrée (21) de l'unité de traitement du sang (20) étant raccordées chacune à une ouverture respective des ouvertures (71-76).

28. Dispositif pour le traitement du sang selon la revendication 27, la valve (6) comprenant deux autres ouvertures (77, 78), la sortie (22) de l'unité de traitement du sang (20) et une sortie du sang (322) du dispositif pour le traitement du sang étant raccordées chacune à une ouverture respective des autres ouvertures (77-78).

29. Dispositif pour le traitement du sang selon la revendication 19 et 27 ou 28, le siège de valve (70) comprenant les six ouvertures (71-76).

30. Dispositif pour le traitement du sang selon l'une quelconque des revendications 18 à 29, la valve (6) étant configurée pour :
séparer la seconde pompe (50) de la ligne de retour du sang (12), diviser la ligne de retrait du sang (11) en une première ligne d'embranchement (13) qui comporte la première pompe à sang (40) et en une seconde ligne d'embranchement (14) raccordée en parallèle avec la première ligne d'embranchement (13), et raccorder la seconde pompe à sang (50) dans la seconde ligne d'embranchement (14), quand la valve (6) est soit dans une première position (DN-P1) soit dans une seconde position (DN-P2),
ouvrir une partie amont (15) et fermer une partie aval (16) de la première ligne d'embranchement (13), et fermer une partie amont (17) et ouvrir une partie aval (18) de la seconde ligne d'embranchement (14) quand la valve (6) est déplacée dans la première position (DN-P1), et
fermer la partie amont (15) et ouvrir la partie aval (16) de la première ligne d'embranchement (13), et ouvrir la partie amont (17) et fermer la partie aval (18) de la seconde ligne d'embranchement (14) quand la valve (6) est déplacée dans la seconde position (DN-P2).

31. Dispositif pour le traitement du sang selon l'une quelconque des revendications 18 à 30, la valve (6) comprenant un conduit (63) configuré pour séparer la seconde pompe (50) de la ligne de retour du sang (12).

32. Dispositif pour le traitement du sang selon l'une quelconque des revendications 18 à 31, l'unité de traitement du sang (20) présentant une forme allongée, la valve (6) étant disposée sur un côté long (9) de l'unité de traitement du sang (20).

33. Dispositif pour le traitement du sang selon l'une quelconque des revendications 18 à 32, la première pompe (40) et la seconde pompe (50) étant disposées chacune sur un côté long (9) de l'unité de traitement du sang (20).

34. Dispositif pour le traitement du sang selon l'une quelconque des revendications 18 à 33, la première pompe (40) et la seconde pompe (50) étant chacune une pompe à membrane.

35. Dispositif pour le traitement du sang, comprenant :
une unité de traitement du sang (20) comportant une entrée (21) et une sortie (22)
une ligne de retrait du sang (33) configurée pour recevoir du sang une ligne de retour du sang (32) configurée pour retourner du sang,
une première pompe (40) comportant une entrée (41) et une sortie (42),
une seconde pompe (50) comportant une entrée (51) et une sortie (52),
**caractérisé par**
un siège de valve (70) comprenant au moins six ouvertures (71-76), l'entrée (21) de l'unité de traitement du sang (20), la ligne de retrait du sang (33), l'entrée (41) et la sortie (42) de la première pompe (40), et l'entrée (51) et la sortie (52) de la seconde pompe (50) étant raccordées chacune à une ouverture respective des six ouvertures (71-76), afin de permettre à un corps de valve (60) disposé dans le siège de valve (70) de contrôler un flux de sang via au moins deux des six ouvertures (71-76).

36. Dispositif pour le traitement du sang selon la revendication 35, le siège de valve (70) comprenant deux autres ouvertures (77, 78), la ligne de retour du sang (32) et la sortie (22) de l'unité de traitement du sang (20) étant raccordées chacune à une ouverture respective des autres ouvertures (77-78).

37. Appareil de traitement du sang comprenant un dispositif pour le traitement du sang (10) selon l'une quelconque des revendications 1 à 36.
